# FASCICULE DE BREVET EUROPEEN

(11) **EP 3 796 862 B1**
(45) Date de publication et mention de la délivrance du brevet: **28.02.2024**
(21) Numéro de dépôt: 19724872.7
(22) Date de dépôt: 22.05.2019
(51) Int. Cl.: A61B 18/20, A61N 5/06

(54) **DISPOSITIF DE TRAITEMENT PAR EMISSION D'IMPULSION LASER**
VORRICHTUNG ZUR BEHANDLUNG MIT LASERPULSEMISSION
DEVICE FOR TREATMENT WITH THE EMISSION OF LASER PULSES

(30) Priorité: 22.05.2018 FR 1854255
(43) Date de publication de la demande: 31.03.2021
(73) Titulaire: Eurofeedback, 91017 Evry Cedex (FR)
(72) Inventeur: SAFRAOUI, Georges, 91140 Villejust (FR)
(74) Mandataire: Cabinet Nony
(86) Numéro de dépôt international: PCT/EP2019/063263
(87) Numéro de publication internationale: WO 2019/224276

(56) Documents cités:
- WO-A1-01/26573
- WO-A2-2010/102197
- US-A1- 2012 232 539
- US-A1- 2014 012 225
- US-A1- 2016 184 017
- US-B1- 6 171 302

## Description

La présente invention concerne les dispositifs de traitement d'une partie d'un corps humain par émission d'impulsions lumineuses comportant une pièce à main reliée à un poste de base, notamment ceux appelés LPP (Lampe Polychromatique Pulsée) ou IPL (Intense Pulse Light) et ceux à rayonnement laser, et les procédés esthétiques de traitement correspondants.

L'invention concerne préférentiellement les traitements esthétiques tels que le photorajeunissement, l'épilation ou le traitement vasculaire.

Les traitements de ce type sont de plus en plus pratiqués par les professionnels au moyen de dispositifs qui se sont très largement démocratisés depuis quelques années et ont pris une place très importante dans les cabinets médicaux et esthétiques.

Ces dispositifs comportent usuellement une pièce à main pour délivrer des impulsions lumineuses à la partie du corps à traiter. La pièce à main présente une fenêtre d'émission des impulsions lumineuses qui ne permet pas de traiter en une seule impulsion une grande partie du corps. Le traitement se fait donc usuellement par un opérateur qui déclenche manuellement une impulsion lumineuse sur une zone de la partie du corps à traiter et déplace la pièce à main pour ensuite déclencher une nouvelle impulsion lumineuse sur une nouvelle zone adjacente à la précédente et ainsi de suite. Une telle méthode est relativement lente et coûteuse.

Il est connu de la demande internationale WO 01/26573 un dispositif de traitement de la peau, notamment d'épilation, par émission d'impulsions laser sur la peau, comportant un système électronique déterminant la position de la pièce à main sur la peau et déclenchant automatiquement l'émission d'une impulsion lumineuse lorsque la zone sur laquelle la pièce à main est disposée est à traiter. Dans ce dispositif, la pièce à main comporte un détecteur des mouvements de la pièce à main se présentant sous la forme d'un rouleau et un détecteur de la position de la pièce à main par rapport à un élément fixe se présentant sous la forme d'un transpondeur et d'un ou plusieurs détecteurs. Le détecteur de mouvement sous forme de rouleau ne permet de détecter les mouvements de la pièce à main que selon une direction. De plus, un tel rouleau n'est pas compatible avec l'application d'un gel sur la peau, qui ne permettrait plus au rouleau de rouler sans glisser sur la peau. Si le corps à traiter bouge, les informations sur les zones restant à traiter sont susceptibles d'être faussées et des zones peuvent ne pas être traitées.

Il est également connu de la demande US 2013/0237973 un dispositif d'épilation par émission d'impulsions laser sur la peau comportant une unité de commande distinguant les zones ayant reçu une émission laser des zones n'en ayant pas reçue à partir d'informations de direction et de distance de mouvement de la pièce à main, obtenues grâce à un détecteur sur la pièce à main couplé ou non à un marquage sur la peau et une caméra externe couplée à un repère sur la pièce à main. L'unité de commande affiche ces informations sur un organe d'affichage de sorte que l'opérateur puisse identifier les zones épilées ou non encore épilées, déplacer la pièce à main et actionner les impulsions lumineuses en conséquence. Différents types de détecteurs sont décrits. Ce dispositif nécessite que l'opérateur déclenche manuellement chaque impulsion lumineuse.

US 2016/184017 divulgue un dispositif de photorajeunissement par émission d'impulsions lumineuses.

Il existe donc un besoin pour bénéficier d'un dispositif permettant un traitement, notamment de photo-rajeunissement ou d'épilation, ou un traitement vasculaire, sûr, fiable, précis et rapide.

### Résumé

L'invention répond à ce besoin, à l'aide d' un dispositif de traitement d'une partie d'un corps humain par émission d'impulsions lumineuses, comportant :
o une pièce à main pour appliquer les impulsions lumineuses sur la partie du corps, la pièce à main étant déplaçable par rapport au corps et comportant une fenêtre de sortie des impulsions lumineuses sur une zone localisée de la partie du corps, et
o au moins un système de projection pour projeter une information de guidage sur la partie du corps afin de guider l'opérateur durant le traitement et/ou une information d'aide à la localisation de la pièce à main et/ou du corps, le système de projection éclairant au moins partiellement la partie du corps à traiter pour différencier les zones traitées des zones non traitées de la partie du corps à traiter.

Le dispositif peut comporter une ou une combinaison des caractéristiques suivantes :
- Le traitement est non invasif. De préférence, le traitement est non chirurgical.
- Le traitement est esthétique.
- La partie du corps à traiter est une zone de la surface de la peau.
- La fenêtre de sortie des impulsions lumineuses est configuré pour ne permettre l'émission des impulsions lumineuses que sur une seule zone à une position de la pièce à main donnée,
- L'information de guidage de la partie du corps est une information relative aux zones de la partie du corps à traiter non traitées.
- Le système de projection éclaire au moins partiellement la partie du corps à traiter pour différencier, notamment par une différence de luminosité, couleur ou éclairage, les zones traitées des zones non traitées de la partie du corps à traiter.
- Le système de projection produit une image sur la partie du corps configurée pour différencier, notamment par une différence de luminosité, couleur, les zones de la peau par le nombre de passage de la pièce à main qu'elle ont subi.
- La projection de l'information sur la zone de la partie du corps par le système de projection est ajustée en temps réel en fonction des mouvements du corps.
- Le dispositif comporte deux systèmes de projection orientées selon des directions de visée différentes par rapport à la partie du corps à traiter.
- Le système de projection projette des franges sur la partie du corps pour aider à la détermination des mouvements de la partie du corps.
- Le dispositif comporte une caméra d'analyse des franges projetées sur la peau.
- La pièce à main peut comporter un détecteur de mouvement, notamment optique, capacitif ou inertiel.

Le dispositif peut comporter un système de commande de l'émission des impulsions lumineuses configuré pour déclencher automatiquement les impulsions lumineuses lors du déplacement de la pièce à main à une cadence déterminée en fonction au moins d'au moins une information fournie par le détecteur de mouvement.

L'invention a encore pour objet, un procédé esthétique de traitement d'une partie d'un corps par émission d'impulsions lumineuses à l'aide d'un dispositif tel que décrit ci-haut, comportant les étapes consistant à projeter sur la partie du corps un information de guidage ou d'aide à la localisation de la pièce à mainavec le système de projection et à traiter la partie du corps en fonction de l'information de guidage et/ou à localiser la pièce à main et/ou le corps.

Le procédé peut comporter une ou une combinaison des caractéristiques suivantes :
- Le procédé peut comporter une étape d'application d'un gel sur la peau de la partie du corps à traiter et/ou sur la pièce à main,
- Le procédé peut comporter une étape d'application de la pièce à main sur la partie du corps à traiter et une étape de balayage de la partie du corps à traiter avec la pièce à main, notamment en maintenant une pression avec la pièce à main sur la peau.

Le système de commande peut déclencher automatiquement les impulsions lumineuses lors du déplacement de la pièce à main sur la peau.

L'invention est définie dans les revendications attenantes.

L'invention pourra être mieux comprise à la lecture de la description qui va suivre, d'exemples de mise en oeuvre non limitatifs de celle-ci, et à l'examen du dessin annexé, sur lequel :
- La figure 1 illustre de manière schématique une séance de traitement à l'aide d'un dispositif selon la description, vu du dessus,
- La figure 2 est une représentation schématique en perspective de la pièce à main du dispositif de la figure 1,
- Les figures 3A à 3C illustrent l'agencement des zones ayant reçu des impulsions lumineuses successives lors de la séance de traitement de la figure 1,
- La figure 4 représente la séance de traitement de la figure 1 selon une autre vue,
- La figure 5 illustre schématiquement un exemple de système optique d'un dispositif selon la description,
- La figure 6 est une vue schématique en coupe d'une pièce à main d'un dispositif selon la description,
- La figure 7 représente schématiquement une variante de pièce à main selon la description,
- Les figures 8A et 8B représentent des variantes de balayage de la partie du corps à traiter avec un dispositif selon la description,
- La figure 9 illustre schématiquement l'utilisation d'un système de visualisation sur le corps humain du traitement à effectuer,
- La figure 10 représente schématiquement un exemple de détecteur de mouvement,
- Les figures 11A à 11C représentent des séquences de déclenchement des rangées de diodes laser d'un dispositif selon la description,
- La figure 12 est une représentation schématique d'un système de focalisation réglable d'un dispositif selon la description, et
- La figure 13 est un exemple d'une courbe de décroissance de température limite de la peau.

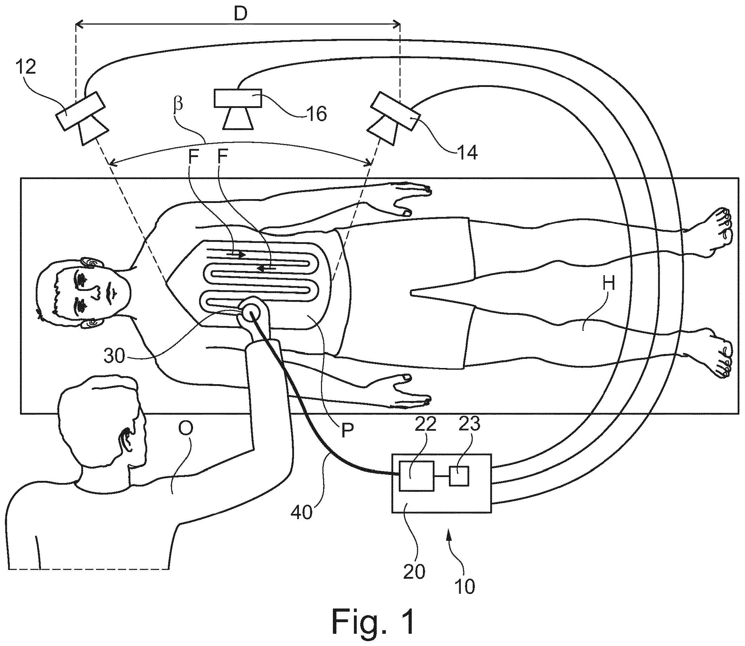

La séance de traitement représentée sur la figure 1 est une séance d'épilation effectuée par un opérateur O sur une partie à traiter P d'un corps humain H à l'aide d'un dispositif 10 comportant un poste de base 20, une pièce à main 30 déplaçable sur le corps H, trois caméras 12, 14 et 16 chacune reliée au poste de base 20. La caméra 16 est une caméra thermique.

Le poste de base 20 comporte une source laser 22 et un système de refroidissement de la source laser, non représenté.

La pièce à main 30 est reliée au poste de base par au moins un guide optique souple 40 permettant le transport des impulsions laser émises par la source laser 22 à la pièce à main 30.

Le fait que la source laser 22 soit déportée de la pièce à main 30 permet de s'affranchir des contraintes de taille ou de poids du système de refroidissement, ce qui permet d'avoir un système de refroidissement de la source laser 22 plus efficace que s'il était dans la pièce à main 30. Ceci permet également d'avoir une pièce à main plus légère et donc plus maniable.

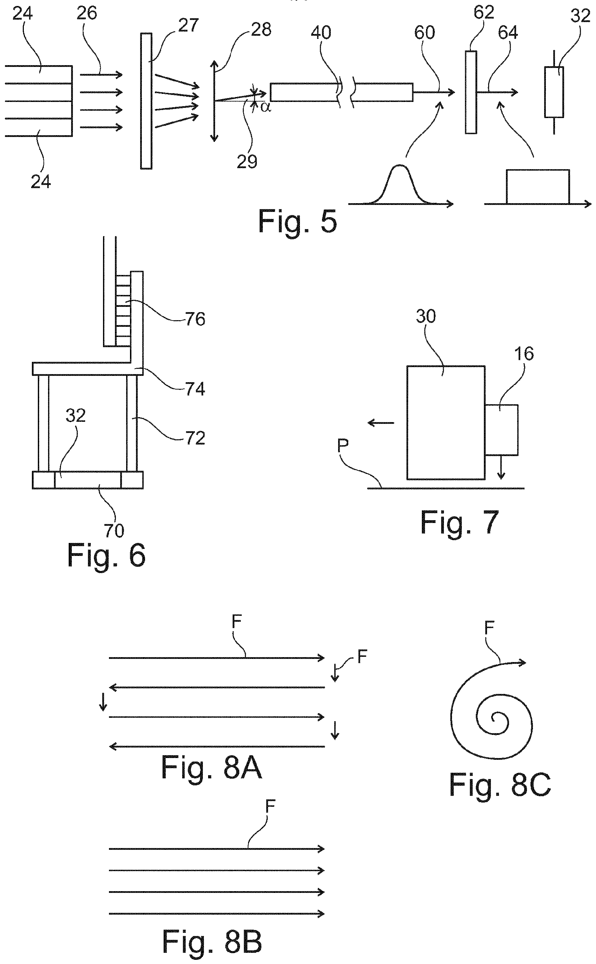

Comme cela est illustré sur la figure 5, la source laser 22 comporte une pluralité de diodes laser agencées selon plusieurs rangées 24, par exemple des diodes laser agencées selon 4 rangées de 10 à 30 diodes laser chacune. Les diodes laser d'une rangée sont montées en série et les rangées sont soit montées en parallèles les unes par rapport aux autres soit commandées indépendamment entre elles.

Dans chaque rangée 24, au moins l'une des diodes laser émet dans le visible à une longueur d'onde visible par l'opérateur O durant le traitement et les autres diodes émettent toutes soit à 750 nm, soit à 1064 nm. De la sorte, les composantes à 750 nm ou 1064 nm de chaque rangée 24 permettent l'épilation et la composante dans le visible permet que les impulsions laser soient visibles, ce qui permet de faciliter le suivi de l'épilation par l'opérateur O et d'accroître la sécurité pour ce dernier. La partie de base présente au moins une rangée 24a émettant à 750 nm et au moins une rangée 24b émettant à 1064 nm. Il est possible de faire varier l'intensité des impulsions laser émises par une rangée 24 en faisant varier le courant d'alimentation de cette dernière. Ceci permet une grande modularité de traitement en fonction des caractéristiques du corps à traiter en permettant de choisir à la fois les rangées 24a et/ou 24b à déclencher, leurs intensités I₁ et/ou I₂ et la séquence de déclenchement de chacune des rangées 24a ou 24b. En effet, la longueur d'onde à 750 nm est bien adaptée à l'épilation des phototypes clair notamment I à IV et la longueur d'onde à 1064 nm est bien adaptée à l'épilation des phototypes foncés notamment V et VI.

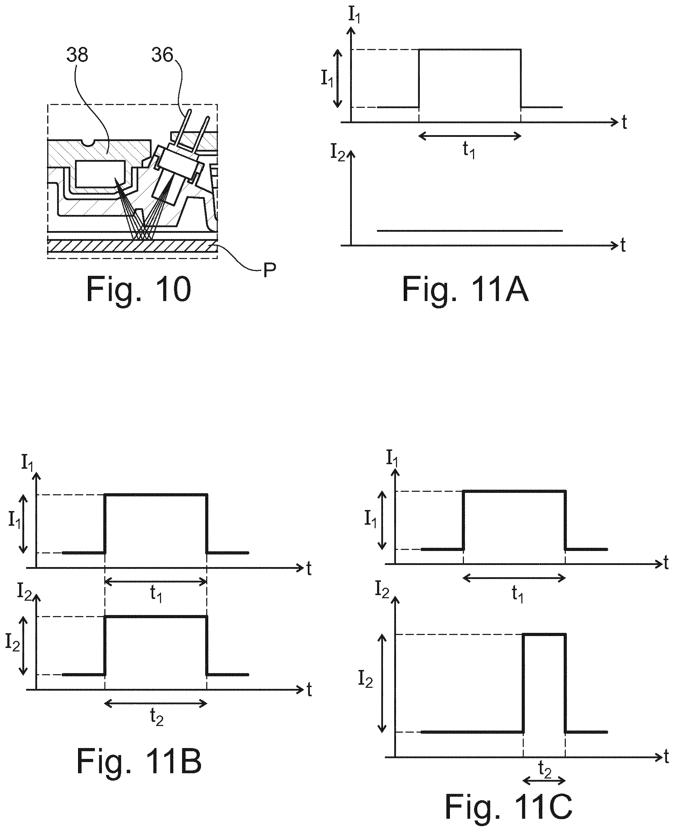

Par exemple, il est possible de déclencher une rangée 24a ou 24b à une certaine intensité I₁ ou I₂ et pendant une certaine durée t₁ ou t₂ comme cela est illustré sur la figure 11A, ou de déclencher simultanément des rangées différentes 24a et/ou 24b à une même ou une intensité différente I₁ et I₂ respectivement et pendant une même durée t₁ et t₂ respectivement, comme cela est illustré sur la figure 11B, où il est possible de déclencher une ou plusieurs rangées 24a émettant à 750 nm à une intensité I₁ sur une première durée t₁ et de déclencher une ou plusieurs rangées 24b émettant à 1064 nm avec une intensité plus grande I₂ mais sur une deuxième durée t₂ plus courte, la ou les rangées 24b étant déclenchées de sorte que leur émission laser se termine en même temps que l'impulsion laser émise par les rangées 24a, comme cela est illustré sur la figure 11C Les faisceaux émis par les diodes laser 26 sont concentrés par une lentille 27 comportant une pluralité de micro lentilles formées sur un support, les microlentilles étant configurées pour faire converger les faisceaux des diodes laser 26 vers une lentille 28. Cette dernière est disposée pour faire rentrer le faisceau résultant 29 dans le guide optique 40 avec un angle d'incidence adéquat.

De préférence, les impulsions laser entrant dans le guide optique présentent une durée comprise entre 1 ms et 100 ms, mieux entre 5 ms et 20 ms.

De préférence, le guide optique 40 est une guide optique liquide. Le guide optique 40 est configuré pour faire passer un rayonnement laser présentant une intensité de plusieurs kW en mode continu. En variante, le guide optique est une fibre optique ou un faisceau de fibres optiques.

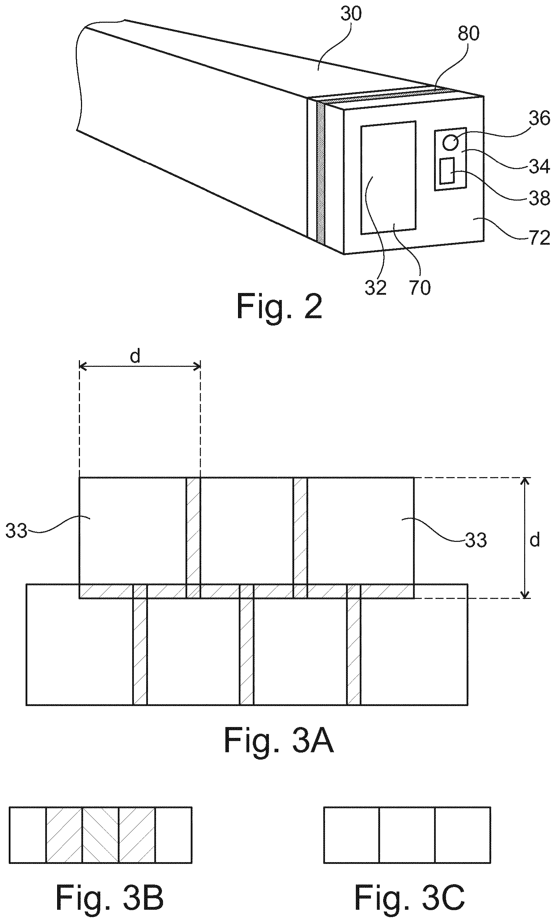

Comme cela est illustré sur la figure 2, la pièce à main 30 comporte une fenêtre de sortie 32 des impulsions laser, de section polygonale, notamment carrée ou rectangulaire, présentant un saphir 70, de préférence refroidi. De préférence, la fenêtre de sortie 32 est totalement étanche au liquide. La fenêtre de sortie 32 est entourée par une semelle métallique 72, destinée à venir au contact de la peau durant le traitement. La forme polygonale, notamment carré de la fenêtre de sortie permet l'optimisation du nombre de flashs et d'éviter un trop grand recouvrement de flashs.

En sortie du guide optique 40, la pièce à main 30 comporte un système optique 62 permettant de transformer la section du faisceau, qui est généralement sensiblement circulaire en sortie du guide optique 40, en une section du faisceau émis présentant une forme sensiblement rectangulaire sans qu'il n'y ait perte d'énergie, de préférence de forme sensiblement carrée, tel qu'illustré sur la figure 3. Le système optique 62 peut également permettre d'homogénéiser la distribution de la lumière du faisceau en transformant la distribution gaussienne de la lumière en une distribution carrée. La distribution de la lumière du faisceau en sortie du système optique 64 est ainsi homogène sur toute sa section. Ceci permet de limiter les risques de brûlures localisées de la peau.

Le système optique 64 correspondant comporte une lentille présentant une pluralité de micro lentilles formées sur un support permettant de transformer la section du faisceau, qui est généralement sensiblement circulaire en sortie du guide optique 40, en une section du faisceau émis présentant une forme sensiblement rectangulaire sans qu'il n'y ait perte d'énergie, et un cristal configuré pour homogénéiser la distribution de la lumière du faisceau.

Un tel système optique permet qu'une impulsion laser couvre une zone localisée 33 du corps de même forme que la fenêtre de sortie 32, i.e. de forme polygonale, notamment carrée ou rectangulaire, ce qui permet, en déplaçant la pièce à main 30 sur le corps H, de juxtaposer facilement deux zones 33 sans qu'il n'y ait d'espace entre les deux zones adjacentes. Les deux zones 33 adjacentes sont jointives et peuvent se recouper au moins partiellement, comme cela est illustré sur les figures 3A ou 3B, ou non, comme cela est illustré sur la figure 3C. Ceci permet un traitement plus efficace en limitant les risques que certaines zones ne soient pas traitées. L'invention n'est pas limitée à une section de sortie des impulsions laser de forme carrée. Ce qui importe, c'est que la section de sortie des impulsions laser présente deux bords opposés parallèles permettant de quadriller la partie du corps P selon un pavage dépourvu d'espace entre les zones 33 adjacentes.

Les deux zones 33 adjacentes peuvent se recouper sur 50 % de leur largeur d, comme cela est illustré sur la figure 3A. De la sorte, chaque zone de la peau reçoit deux impulsions laser successives. Dans la variante illustrée sur la figure 3B, les deux zones adjacentes se recoupent sur une petite portion de leur largeur d, par exemple inférieure ou égale à 20 %, mieux inférieure ou égale à 10 %. Ce léger recoupement permet d'éviter que certaines zones de la peau ne soient pas traitées entièrement. En variante, comme illustré sur la figure 3C, les deux zones adjacentes se juxtaposent sans se superposer.

Comme cela est illustré sur la figure 6, le saphir 70 peut être refroidi par un système de refroidissement comportant des caloducs 72 reliant la semelle 72 de la pièce à main comportant le saphir 70 à une pièce 74 en un matériau de bonne conductivité thermique, par exemple en aluminium, elle-même en contact avec des cellules à effet Peltier 76.

Dans une variante non illustrée, le saphir 70 est refroidi uniquement par les caloducs reliés à une pièce métallique reliés à un liquide de refroidissement, notamment de l'eau ou la semelle 72 de la pièce à main portant le saphir est en un matériau de bonne conductivité thermique, notamment de l'aluminium, reliée directement sans caloducs à des cellules à effet Peltier.

En sortie de la pièce à main 30, chaque impulsion laser présente une puissance surfacique maximale supérieure ou égale à 1 kW/cm². Une telle puissance permet une bonne efficacité d'épilation du corps même sur les poils fins.

En fonction des propriétés de la peau, et notamment du phototype de la peau, l'opérateur peut moduler l'énergie et la puissance des impulsions laser avant le traitement ou pendant le traitement. Ceci évite de brûler la peau.

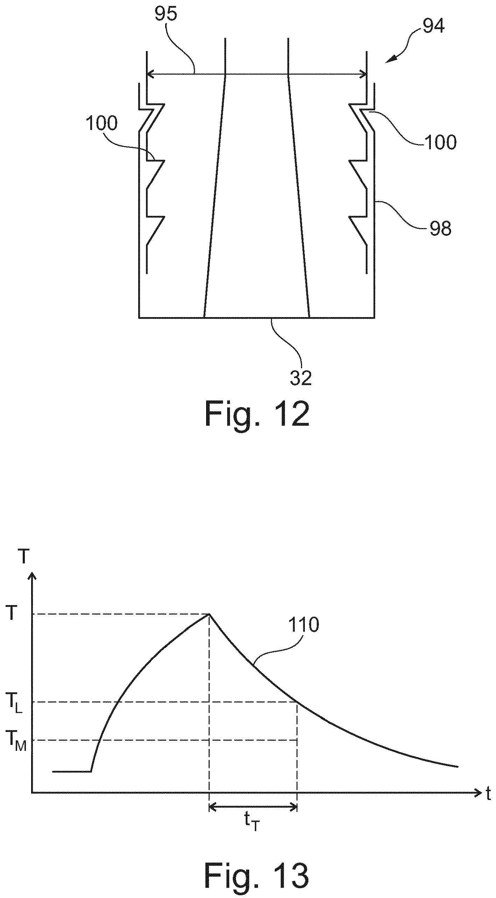

La pièce à main peut également comporter un système de focalisation réglable 94 représenté sur la figure 12 permettant de modifier la focalisation de l'impulsion laser en sortie de la pièce à main 30. Un tel système permet à l'opérateur O de concentrer plus ou moins le faisceau laser dans une zone bien précise. Le système de focalisation 94 est formé d'une lentille divergente 95 rendant le faisceau de l'impulsion laser conique et d'une portion télescopique 98 de la pièce à main 30 permettant de changer la distance entre la lentille 95 et la fenêtre de sortie 32. Ainsi, en allongeant la portion télescopique 98 de la pièce à main, l'opérateur O peut agrandir la section du faisceau ou inversement. La portion télescopique 98 de la pièce à main comporte des reliefs d'encliquetage et des reliefs correspondants 100 pour bloquer la portion télescopique dans certains allongements prédéfinis. La pièce à main 30 comporte un détecteur de l'allongement de la portion télescopique 98 non représenté. Le dispositif peut alors détecter l'allongement prédéfinie de la pièce à main dans lequel la pièce à main est réglée, en déduire la focalisation et changer automatiquement les paramètres des impulsions lumineuses selon les paramètres d'impulsions lumineuses particuliers associés à l'allongement prédéfini choisi par l'opérateur. Une telle focalisation réglable permet de régler la dimension surfacique de la surface traitée et la puissance des impulsions lumineuses en fonction d'au moins une caractéristique de la zone à traiter. Par exemple, dans le cas de poils de large diamètre, l'opérateur peut choisir d'allonger la pièce à main 30 pour élargir la largeur du faisceau et traiter une zone large à chaque impulsion laser. En effet, dans le cas de poils de large diamètre, l'épilation est efficace avec une puissance surfacique plus faible que dans le cas de poils fins. Le traitement est d'autant plus rapide que la section du faisceau en sortie est large.

Comme cela est illustré sur la figure 2, la pièce à main 30 comporte également un détecteur de mouvement 34 optique ou inertiel, permettant de détecter les mouvements de la pièce à main 30 selon au moins deux directions. Le détecteur de mouvement 34 est fixe par rapport à la pièce à main. De préférence, le détecteur de mouvement 34 comporte une source lumineuse 36, tel qu'une LED ou une diode laser, et un détecteur optique 38 multi-pixel, par exemple une micro-caméra, permettant de détecter la lumière issue de la source lumineuse 36 et réfléchie par la peau, comme cela est illustré sur la figure 10. L'analyse de la lumière reçue par la détecteur optique 38 permet de déduire des informations de mouvement de la pièce à main 30 sur la peau lorsque cette dernière est déplacée en restant en contact avec la peau. Il est alors possible de déclencher automatiquement les impulsions laser successives en fonction des mouvements de la pièce à main 30 sur la peau, comme cela sera détaillé ci-dessous. Les zones traitées peuvent donc être réparties régulièrement sur la partie du corps à traiter.

De préférence, le détecteur optique 38 présente une résolution supérieure ou égale à 100 pixels, mieux supérieure ou égale à 250 pixels, encore mieux supérieure ou égale à 500 pixels, par exemple égale à 900 pixels. De préférence, le détecteur optique 38 prend plus de 1000 images/s, mieux plus de 1500 images/s, encore mieux plus de 3000 images/s, par exemple 6400 images/s.

De préférence, le détecteur optique 38 prend une image de la peau pendant une durée inférieure ou égale à 500µs, mieux inférieure ou égale à 300 µs.

De préférence, les pixels de la caméra imagent chacun une zone de la peau de largeur inférieure ou égale à 100 µm, mieux inférieure ou égale à 50 µm encore mieux inférieure ou égale à 20 µm. Une telle résolution permet en analysant les images prises par le détecteur optique 38 de déterminer le diamètre des poils. La source lumineuse 36 peut éclairer la peau en continu. En variante, elle éclaire en discontinu la peau avec une durée d'éclairage très courte. L'éclairage peut être stroboscopique et de durée inférieure à 300µs mieux inférieur à 100µs.

Un tel détecteur de mouvement 34 permet de détecter un mouvement inférieur ou égal à 1 mm, mieux inférieur ou égal à 0,8 mm, encore mieux inférieur ou égale à 0,5 mm.

Le détecteur optique 38 peut être une micro-caméra qui permet de former une image de la peau, similaire aux détecteurs équipant les souris d'ordinateurs.

Le dispositif comporte un système de traitement des images fournies par le détecteur optique 38. Un tel système peut détecter les poils dans lesdites images. Il peut alors déterminer, en connaissant la position de la pièce à main sur la peau, la localisation, le diamètre de chaque poil imagé et/ou le nombre de poils. A partir de ces informations, il est possible d'estimer la quantité de poils sur la partie du corps à traiter et/ou leurs diamètres associés et/ou la distribution des poils sur la partie du corps. Cette détermination peut se faire même si la surface détectée par la micro-caméra 38 est plus petite que la dimension surfacique de la fenêtre 32. En effet, dans ce cas, il est possible d'extrapoler la distribution des poils sur toute la partie du corps à partir de la distribution des poils déterminée sur la portion du corps imagée par la micro-caméra 38. Ceci permet d'adapter les paramètres du traitement en fonction de la densité de poils sur la zone à traiter. Cette estimation peut se faire indépendamment de l'émission des impulsions laser par la pièce à main. Elle peut alors être réalisée pendant le traitement, lors du balayage de la peau avec la pièce à main 30, ou indépendamment du traitement en balayant la peau avec la pièce à main 30 sans émettre les impulsions laser.

Tant que la pièce à main 30 reste en contact avec la peau, le détecteur de mouvement 34 peut permettre de connaitre la position de la pièce à main 30 par rapport à sa position initiale avec une précision de l'ordre du millimètre.

Un tel détecteur de mouvement 34 permet également de donner des informations relatives à l'appui ou non de la pièce à main 30 sur la peau. En effet, lorsque la pièce à main 30 n'est plus en contact avec la peau, le détecteur optique 38 ne permet plus de recevoir une image nette de la peau. L'émission des impulsions laser peut être arrêtée automatiquement lorsque la pièce à main 30 n'est pas en contact avec la peau. Un avertisseur visuel ou sonore peut également être prévu, par exemple une petite diode ou une fenêtre d'information sur un écran 50, permettant de signaler à l'opérateur O que la pièce à main n'est plus en contact avec la peau et qu'aucune impulsion laser n'est émise.

Dans une variante non illustrée, le détecteur de mouvement 34 comporte trois sources lumineuses 36 de couleurs différentes, notamment rouge, verte et bleue. Les sources lumineuses émettent successivement une lumière. Chaque émission est détectée par la détecteur optique 38 et une image couleur de la peau est recomposée à partir des images prises par le détecteur optique 38 pour chacune des longueurs d'ondes. Les sources lumineuses peuvent être trois diodes laser ou trois LEDs. En variante, les trois sources lumineuses sont intégrées dans une seule diode laser ou une seule LED.

Dans ces cas où une image en couleur est formée, le détecteur de mouvement 34 est de préférence positionné en amont de la fenêtre de sortie 32 des impulsions laser eu égard à la direction de déplacement de la pièce à main de sorte à permettre l'obtention d'une image de la peau en couleur avant qu'elle ne soit soumise à une impulsion laser. Les caractéristiques des impulsions lumineuses peuvent alors être changées automatiquement en fonction des caractéristiques locales de la peau. Par exemple, le dispositif peut changer automatiquement les caractéristiques des impulsions laser lorsque la pièce à main 30 passe d'une zone bronzée à une zone non bronzée. Une telle variante impose que la pièce à main soit toujours orientée de la même manière lors du déplacement de la pièce à main sur la peau.

En variante encore, le détecteur de mouvement 34 peut être un détecteur capacitif multi-pixel permettant d'imager les microreliefs de la peau ou un accéléromètre.

La fréquence de cadence maximale de tir des impulsions laser est de préférence supérieure ou égale à 30 Hz, mieux 50 Hz. De telles fréquences maximales combinées avec un déclenchement automatique des impulsions laser permettent de traiter rapidement la partie du corps à traiter.

Les caméras 12 et 14 sont reliées électriquement au poste de base et sont disposées de sorte à observer chacune l'intégralité de la partie du corps à traiter P. Les deux caméras 12 et 14 sont disposées selon deux orientations différentes de sorte à avoir des directions de visée différentes. Elles permettent de visualiser la partie du corps et de localiser la pièce à main 30 sur ce dernier. Elles forment entre elle un angle β compris entre 45 et 120, par exemple 90°. Un système de traitement des images fournies par les caméras 12 et 14 permet d'avoir une visualisation en trois dimensions de la partie du corps à traiter. Ceci est particulièrement utile dans le cas où l'on traite une partie du corps telle que les bras ou les jambes.

Comme cela est illustré sur la figure 2, la pièce à main 30 peut comporter un marquage 80 permettant son repérage par les caméras 12 et 14. De préférence, ce marquage 80 s'étend selon au moins deux directions de sorte à permettre la reconnaissance de la pièce à main 30 par les deux caméras 12 et 14. Le marquage 80 peut être un marquage visuel, par exemple un trait noir, ou un ou plusieurs repères lumineux sur la pièce à main 30, par exemple un halo lumineux ou des LED. Il est alors possible de déterminer la position de la pièce à main 30 sur la partie du corps à traiter avec une précision de l'ordre de quelques millimètres. Un tel repérage de la pièce à main 30 relativement à la partie du corps à traiter permet en particulier un suivi du traitement en toute circonstance, notamment lorsque l'opérateur a soulevé la pièce à main 30 de la peau ou en l'absence de la détection du mouvement par le détecteur de mouvement. En effet, dès que l'opérateur soulève la pièce à main 30, le détecteur de mouvement 34 ne permet plus de suivre les mouvements de la pièce à main 30 sur la peau. Les caméras 12 et 14 permettent de prendre le relais de la localisation de la pièce à main.

Pour affiner la détermination du positionnement de la pièce à main 30 sur la partie du corps, les informations fournies par les caméras 12 et 14 peuvent être couplées avec un système de détermination des mouvements de la partie du corps P pour permettre de prendre en compte les mouvements du corps durant l'épilation, notamment dans la détermination précise de la position de la pièce à main 30 et la détermination des zones du corps traités par la pièce à main 30. Le système de détermination des mouvements peut être un modèle numérique du corps humain en trois dimensions à calibrer en fonction du corps à traiter P. Un tel modèle permet, en fonction des informations fournies par les caméras 12 et 14, de calculer facilement les mouvements du corps sans avoir à analyser les images des caméras pixels par pixels, ces derniers étant précalculés dans le modèle.

En variante, le système de détermination des mouvements comporte un repère visuel sur la peau, par exemple une délimitation de la partie du corps à traiter, ou mieux un marquage, notamment un quadrillage sur la partie du corps à traiter, des formes géométriques espacées, notamment régulièrement réparties sur la partie du corps à traiter, par exemple des points, traits, croix ou tout autre repérage. Le marquage peut être imprimé avec une encre, de préférence qui laisse passer la ou les longueurs d'ondes des impulsions lumineuses, notamment fluorescente, et transparente aux infrarouges. Le système de traitement des images de la ou des caméras peut alors déterminer les mouvements du corps en fonction des déformations du quadrillage ou des mouvements des formes les unes par rapports aux autres qu'il détecte.

En variante encore, le système de détermination des mouvements de la partie du corps comporte une projection de franges sur la partie du corps, notamment à l'aide du système de projection précédemment décrit et d'au moins une caméra, notamment la ou les caméras de détection de la pièce à main sur la partie du corps ou d'un projecteur additionnel, notamment un projecteur laser et/ou d'une caméra additionnelle. L'analyse des déformations des franges permet alors de déduire les mouvements de la partie du corps.

Le système de détermination des mouvements de la partie du corps effectue une analyse des mouvements de la partie du corps toutes les t millisecondes, t étant inférieur ou égal à 20 ms, par exemple égal à 15 ms.

L'ensemble des informations fournies par le détecteur de mouvement 34 et les caméras 12 et 14 ci-dessus sont transmises à un système de commande non illustré, configuré pour traiter ces informations et déclencher automatiquement les impulsions laser de la source laser 22 lors du déplacement de la pièce à main 30 relativement au corps H à une cadence déterminée en fonction des informations fournies par le détecteur de mouvement. La cadence est déterminée de telle sorte que la distance parcourue par la pièce à main entre deux impulsions laser soit inférieure ou égale à d, où d est la dimension de la fenêtre de sortie dans la direction de déplacement de la pièce à main, de sorte que les zones soumises aux impulsions laser successives se superposent ou non comme cela est illustré sur les figures 3A à 3C et décrit précédemment. Les impulsions laser sont déclenchées automatiquement, ce qui limite le risque d'oubli de certaines zones et permet de traiter rapidement la peau en déplaçant la pièce à main sur celle-ci sans avoir à se soucier de déclencher les impulsions.

La caméra thermique 16 est également reliée électriquement au poste de base 10. Elle fournit des images du profil de température de la partie à traiter. Les images fournies par la caméra thermique 16 permettent de déduire la température locale de la zone exposée à l'impulsion laser dès que ladite zone n'est plus cachée par la pièce à main 30. De ce fait, la mesure de température est effectuée un certain temps t_{T} après l'émission de la ou des impulsions lumineuses correspondantes. Ce temps t_{T} peut être calculé à partir de la vitesse de la pièce à main 30 sur la peau. Le dispositif compare alors, comme cela est illustré sur la figure 13, la température mesurée T_{M} à une température limite T_{L} mesurée sur une courbe de décroissance limite 110 au temps t_{T} et si la température mesurée T_{M} est supérieure à la température limite T_{L}, le dispositif arrête le traitement ou prévient l'opérateur O que la température de la peau est trop élevée et qu'il y a des risques de brûlure de cette dernière. La température limite T_{L} ne doit, dans tous les cas, pas dépasser 40°C.

En mémorisant pour chaque zone du corps les informations fournies par la caméra thermique 16, le système de commande peut différencier les zones de la partie à traiter P qui ont été déjà traitées des zones non encore traitées. En effet, les zones de la partie du corps traitées ont, à un moment du traitement, subi une élévation de leur température dû aux impulsions lumineuses qu'elles ont reçues et au cours du traitement, le détecteur de température a mesuré au moins une fois cette élévation de température.

Dans la variante illustrée sur la figure 7, la caméra thermique 16 est disposée sur la pièce à main 30, notamment en aval de la fenêtre de sortie 34, de sorte que la caméra thermique visualise la zone de la peau ayant immédiatement reçu une impulsion lumineuse lors du déplacement de la pièce à main. Une telle variante impose que la pièce à main soit toujours orientée de la même manière relativement à la direction de déplacement de la pièce à main sur la peau. Dans ce cas, il est préférable que la caméra thermique 16 soit éloignée de la peau.

A partir des données des déplacements de la pièce à main 30 déterminées par les caméras 12 et 14 et/ou par le détecteur de mouvement 36 et/ou des informations de température données par la caméra thermique 16, le système de commande 23 peut déterminer le nombre de passages de la pièce à main 30 sur chacune des zones ayant de la partie du corps. Le système de commande 23 peut alors bloquer l'émission d'une impulsion laser lorsque la pièce à main 30 est disposée sur une zone sur laquelle elle est déjà passée un nombre de fois prédéterminé.

A partir des données des déplacements de la pièce à main 30 déterminées par les caméras 12 et 14 et/ou par le détecteur de mouvement 36 et/ou des informations de température données par la caméra thermique 16, le système de commande 23 peut déterminer le temps depuis le dernier passage de la pièce à main sur une zone localisée de la partie du corps. Le système de commande 23 peut alors bloquer l'émission d'une impulsion laser lorsque le temps entre deux passages de la pièce à main sur une même zone n'est pas suffisant, notamment pour permettre à la peau d'avoir une température suffisamment faible pour éviter qu'elle ne soit brûlée par les impulsions laser. Ce temps est prédéterminé et peut être dépendant d'au moins une caractéristique de la peau, notamment son phototype.

Le nombre de passages par unité de surface et le nombre d'impulsions par passage est déterminé par l'opérateur préalablement au traitement en fonction des caractéristiques des impulsions laser et de la peau. Comme cela été décrit précédemment, les zones successives soumises aux impulsions laser peuvent ne pas ou peu se superposer de sorte que les zones de la peau ne sont soumises chacune qu'à une impulsion laser à chaque passage. En variante, les zones successives peuvent se superposer par exemple de moitié de sorte que les zones de la partie à traiter reçoivent chacune deux impulsions laser à chaque passage. Le choix de l'une ou l'autre de ces configurations peut dépendre de la sensibilité de la peau. Les caractéristiques des poils et le la peau peuvent nécessiter une pluralité de passages de la pièce à main sur chacune des zones. Dans ce cas, les différents passages doivent être espacés d'un temps permettant une bonne décroissance de la température de la peau.

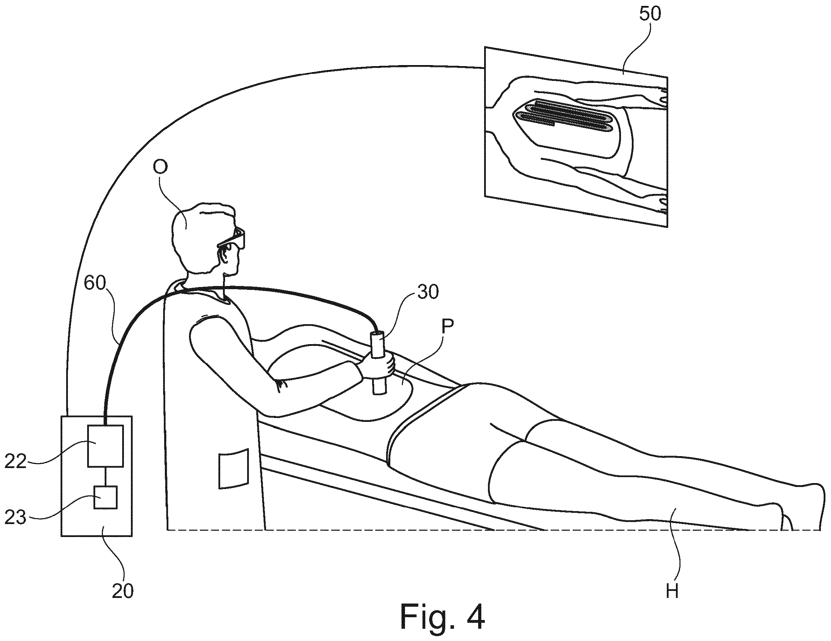

Comme cela est illustré sur la figure 4, le système de commande peut afficher sur l'écran 50 une image de la partie à traiter et différencier sur cette image les zones de la partie à traiter P déjà soumises au nombre prédéterminé d'impulsions laser des autres zones de la partie à traiter. Le système de commande peut également afficher sur l'écran 50 un historique des températures de sorte à permettre un suivi de la température de la peau durant le traitement. L'écran peut être tactile et/ou relié à une souris et un clavier pour permettre à l'opérateur de choisir les paramètres de traitement au début et/ou en cours du traitement, notamment en fonction du phototype de la peau et/ou de la réaction de la peau aux impulsions laser.

De préférence, le système de commande 23 comporte un calculateur pour déterminer notamment les fréquences des impulsions laser, les intensités des impulsions laser et leur puissance en fonction des paramètres choisis par l'opérateur en début de traitement, de la largeur du faisceau d'impulsion laser sélectionnée, de la focale de la pièce à main, des informations reçues par le dispositif en cours de traitement, des mesures effectuées pendant le traitement, notamment de la température mesurée, et de la peau après réception des impulsions laser.

La pièce à main 30 peut comporter un capteur de pression non représenté permettant de mesurer la pression avec laquelle la pièce à main 30 appuie sur la peau. Le système de commande peut ne déclencher les impulsions laser que lorsque la pression détectée par le capteur de pression est supérieure à une pression prédéterminée non nulle. Il peut également être prévu un avertisseur visuel, par exemple une diode ou une fenêtre d'information sur l'écran 50, pour prévenir l'opérateur que la pièce à main n'est pas assez appuyée sur la peau. Le fait d'appuyer la pièce à main 30 sur la peau permet d'améliorer le traitement, notamment dans le cas d'un traitement d'épilation, en chassant le sang sous la peau.

Le détecteur de mouvement peut permettre de détecter la pression exercée par la pièce à main sur la peau en détectant la couleur de la peau sous la pression de la pièce à main. En effet, lors de l'application d'une pression sur la peau, cette dernière change de couleur, notamment blanchie.

Dans le cas de l'épilation, ceci permet de plus de faire ressortir les poils, et ainsi d'améliorer l'efficacité du traitement.

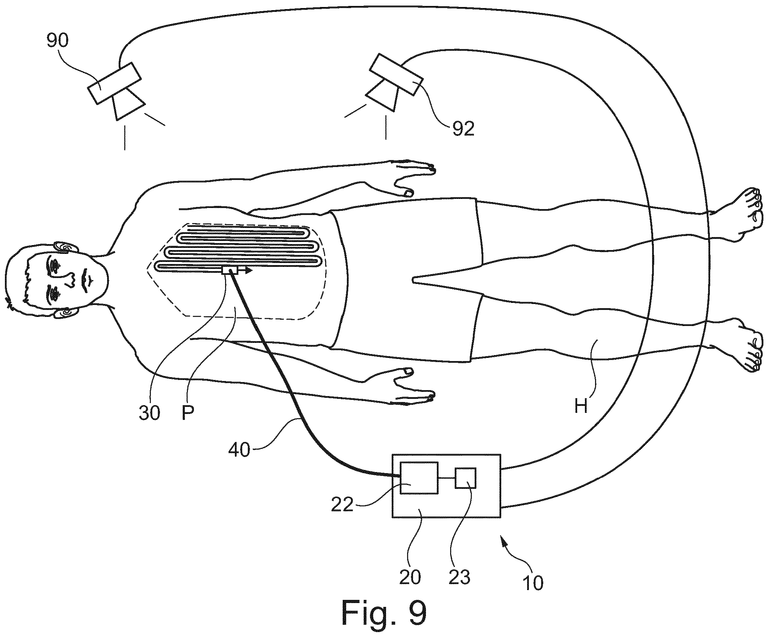

Comme cela est illustré sur la figure 9, le dispositif 10 peut également comporter un système d'information visuelle directement sur la partie du corps P de l'évolution du traitement, par exemple un système permettant de distinguer directement sur la partie du corps les zones ayant déjà été traitées des zones non encore traitées. Un tel système peut par exemple comporter deux projecteurs de lumière 90 et 92 projetant de la lumière sur les zones déjà traitées ou inversement sur les zones non encore traitées. Un tel système peut également distinguer le nombre de passages que chaque zone de la partie du corps à subi, par exemple par différentes couleurs selon le nombre de passages subis. Par exemple, la zone à traiter est affichée en vert par projection d'une image correspondante sur le corps. Au premier passage, la zone traitée apparaît en orange, et au second en rouge.

Le dispositif peut également comporter un dispositif d'enregistrement pour enregistrer le déroulement du traitement. Un tel dispositif d'enregistrement peut permettre d'enregistrer l'image affichée par l'écran au cours du traitement.

Certaines informations prises par le dispositif, notamment l'historique des impulsions lumineuses ou des passages de la pièce à main, le nombre de poils traités et/ou la température cumulée des différentes zones, peuvent être imprimées sur une feuille pour résumer la séance d'épilation.

Le dispositif peut comporter ou être connecté à une base de données clients permettant de conserver d'une séance sur l'autre des informations relatives au traitement, notamment la distribution des poils sur la partie du corps et le nombre de poils mesuré. Ainsi, au fil des séances, l'opérateur O peut déterminer l'historique des séances et avoir une estimation de l'efficacité du traitement, notamment en comparant d'une séance à l'autre la quantité de poils et/ou la distribution des poils sur la partie du corps.

Le dispositif peut aussi comporter un système d'arrêt actionné par la personne recevant le traitement pour arrêter le traitement en cas de douleur ressentie. Un tel système peut constituer une sécurité supplémentaire. L'opérateur peut arrêter le traitement quand il veut.

Le dispositif peut également comporter un sélecteur du mode de fonctionnement pouvant prendre trois modes de fonctionnement différents, un mode automatique dans lequel les impulsions sont déclenchées automatiquement, un mode manuel dans lequel les impulsions sont déclenchées manuellement par l'opérateur O, par exemple à l'aide d'une gâchette sur la pièce à main 30 et un mode neutre sans déclenchement d'impulsions laser.

De préférence, la pièce à main 30 est dépourvue de roulettes. La pièce à main 30 glisse sur la peau par l'application d'un gel sur cette dernière. L'application d'un gel permet de réduire les différences d'indice des interfaces traversées par les impulsions laser, ce qui améliore la transmission d'énergie à la peau. La présence du gel permet également d'éviter tout dégagement de fumée liée à la destruction des poils.

On va maintenant décrire un procédé de traitement par un opérateur O de la partie P du corps à traiter en utilisant un dispositif selon l'invention.

Dans un premier temps, l'opérateur sélectionne les informations relatives au client dans le cas d'un client enregistré sur la base de données ou entre les informations relatives au client dans le cas d'un nouveau client puis détermine un ou plusieurs des éléments suivants :
- la partie du corps à traiter. Il peut par exemple délimiter sur l'image du corps affichée à l'écran la partie du corps à traiter ou encore délimiter directement sur le corps de l'individu, par exemple à l'aide d'un marqueur, la région du corps à traiter. L'opérateur peut également masquer les zones sensibles telles que les grains de beauté e les recouvrant avec un feutre blanc opaque pour les protéger des impulsions laser.
- Le phototype de la peau. L'opérateur O sélectionne le phototype de la peau parmi les phototypes I à VI. Le système de visualisation peut indiquer la couleur de chacun des phototypes pour aider l'opérateur O à choisir le phototype le plus approprié.
- Le nombre N d'impulsions laser par unité de surface lors d'un passage.
- Le nombre p de passages sur chaque unité de surface de la partie du corps.
- La focale en choisissant la longueur de la pièce à main.
- Le diamètre et la couleur des poils parmi une pluralité de couleurs prédéfinies.
- Le mode de fonctionnement manuel ou automatique.

Le calculateur détermine alors les caractéristiques des impulsions laser en fonction des informations fournies par l'opérateur O. Il détermine en particulier le rapport de la distance que doit parcourir la pièce à main entre deux impulsions laser sur la dimension de la section du faisceau dans la direction de déplacement et les séquences de déclenchement des rangées de diodes laser, notamment leurs intensités respectives, leurs durées d'allumage respectives et leurs moments de déclenchement respectifs pour chaque impulsion laser. Le dispositif peut comporter une pluralité de séquences de déclenchement des rangées préenregistrées de sorte que le calculateur sélectionne, en fonction des informations fournies par l'opérateur O la séquence préenregistrée la plus adaptée.

L'opérateur O applique ensuite le gel sur la peau ou la pièce à main 30.

Comme cela est illustré sur les figures 1 et 8A, l'opérateur O applique ensuite la pièce à main 30 sur la peau.

En mode manuel, l'opérateur O positionne la pièce à main et appuie sur la gâchette pour déclencher une impulsion laser puis déplace la pièce à main 30 sur la peau d'une certaine distance et appuie à nouveau sur la gâchette pour déclencher une nouvelle impulsion laser et ainsi de suite. Ce mode est en particulier utilisé pour l'épilation de petites surfaces du visage telles que le philtrum.

En mode automatique, l'opérateur O balaye la partie du corps P avec la pièce à main 30. Il déplace la pièce à main 30 de préférence selon un premier axe parallèle à un des côtés de la fenêtre de sortie 32 des impulsions laser dans une première direction et sur toute la dimension de la partie du corps dans cette direction. Ensuite, il déplace la pièce à main dans une direction perpendiculaire à cette direction principale d'une distance correspondant à la hauteur de la fenêtre de sortie puis déplace la pièce à main selon un deuxième axe parallèle au premier axe dans une direction opposée à la première direction et ainsi de suite. Un tel déplacement permet une bonne couverture de la partie à traiter P. Dans ce schéma de déplacement, la plupart du traitement se fait sans soulever la pièce à main 30. Selon le nombre de passages p sélectionné, l'opérateur O balaye à nouveau la partie du corps P selon le même mouvement ou non. Il est préférable que l'opérateur laisse le temps à la peau de refroidir entre deux passages consécutifs. Il peut par exemple reprendre le balayage au début sans s'arrêter si les zones traitées en début de traitement ont eu le temps de refroidir pendant le balayage, ou attendre quelques secondes avant le passage suivant. Les passages successifs sont espacés d'au moins 1 s, de préférence au moins 2 s, mieux au moins 3 s.

La pièce à main 30 est orientée lors de son déplacement de sorte que la direction de déplacement principale soit parallèle à l'un des côtés de la fenêtre de sortie 32 des impulsions laser.

L'opérateur O déplace la pièce à main sans avoir à se soucier du déclenchement des impulsions laser. Celles-ci sont déclenchées automatiquement par le dispositif et l'opérateur peut suivre le bon déroulement directement à l'écran 50. En temps réel, il visualise directement sur l'écran 50 la position de la pièce à main 30 par rapport aux zones ayant déjà été traitées. A la fin de chaque passage, l'opérateur O peut déplacer la pièce à main 30 directement sur les zones non soumises à une impulsion laser sur ce passage ou passer au passage suivant, l'écran 50 lui permet d'identifier les zones non traitées et le nombre de passages effectué sur chaque zone.

En cas d'alerte du dispositif liée par exemple à une température trop élevée de la peau après l'application d'une impulsion laser, à la perte de contact de la pièce à main avec la peau ou à une vitesse de déplacement de la pièce à main trop rapide, à un changement des caractéristiques de la peau et/ou à une pression de contact de la pièce à main sur la peau trop faible, l'opérateur peut être prévenu pour lui permettre de prendre une mesure appropriée, par exemple modifier les caractéristiques des impulsions laser, repositionner la pièce à main sur la peau, ralentir le déplacement de la pièce à main sur la peau et/ou exercer une pression pus forte sur la peau avec la pièce à main. L'alerte peut se manifester par l'allumage d'une ou plusieurs indications lumineuses, par exemple LEDs, ou par une indication à l'écran 50, par exemple l'ouverture d'une fenêtre temporaire à l'écran. Une telle alerte s'accompagne, dans les cas de la perte de contact de la pièce à main avec la peau et de la pression trop faible, d'un arrêt de l'émission des impulsions laser et peut s'accompagner ou non d'un tel arrêt dans les trois autres cas.

L'opérateur O peut décider à tout moment d'interrompre le traitement en stoppant les mouvements de la pièce à main ou en soulevant cette dernière. L'opérateur peut reprendre le traitement à tout moment à partir de là où il s'était arrêté en suivant les informations de l'écran 50. Lorsque l'opérateur repositionne la pièce à main 30 sur la peau, le dispositif détecte la reprise du traitement et déclenche à nouveau les impulsions laser lorsque la pièce à main est positionnée sur une zone non traitée totalement de la partie du corps et que cette dernière est suffisamment froide.

A la fin du traitement, l'opérateur peut visualiser à l'écran un compte rendu du traitement, notamment l'historique des impulsions laser appliquées que chacune des zones de la partie du corps, les caractéristiques des impulsions lasers appliquées, le nombre de poils traités, la distribution des poils sur la partie du corps, et/ou l'historique de la température du corps cumulée sur l'ensemble du traitement.

Au cours des différentes séances de traitement, l'opérateur peut déterminer l'efficacité du traitement en affichant sur l'écran l'historique de la distribution des poils, leurs diamètres associés et/ou le nombre des poils sur la partie du corps à traiter.

Dans le cas décrit précédemment, la distribution des poils, le diamètre des poils et/ou le nombre de poils sont déterminés durant le traitement. En variante, l'opérateur peut passer le dispositif en mode neutre et balayer la partie du corps avec la pièce à main. En mode neutre, aucune impulsion laser n'est déclenchée. Un tel mode peut permettre d'estimer une cartographie de la peau, et/ou la distribution des poils sur la partie du corps et/ou leurs diamètres associés, et/ou le nombre de poils. Ce mode a pour avantage de pouvoir balayer une partie importante de la surface à traiter, notamment plus de 50% de la surface.

L'opérateur peut déplacer la pièce à main 30 à une vitesse inférieure ou égale à 50 cm/s, de préférence comprise entre 5 et 20 cm/s.

De préférence, l'opérateur O porte des lunettes de protection durant tout le traitement pour filtrer la lumière des impulsions laser et ainsi se protéger les yeux.

Un tel dispositif de traitement et un tel procédé de traitement permettent le traitement d'une surface de 1600 cm², par exemple un dos d'homme, en moins de 5 min, mieux en moins de 2 min, mieux en moins d'1 min.

Dans la variante illustrée sur la figure 8B, l'opérateur O peut déplacer la pièce à main 30 selon des lignes parallèles et selon toujours la même direction. A la fin d'une ligne, l'opérateur O soulève la pièce à main 30 pour venir la repositionner au début de la ligne suivante. Dans ce schéma de déplacement, il est nécessaire de soulever la pièce à main 30. Ce mouvement est particulièrement adapté dans le cas où le détecteur de mouvement 34 présente trois diodes laser de couleurs différentes et/ou que la caméra thermique 16 est sur la pièce à main 30, comme décrit précédemment. Cela permet également de traiter une zone mieux refroidie à chaque nouveau balayage. Les caméras 12 et 14 permettent de connaitre la position de la pièce à main 30 par rapport au corps en permanence.

L'invention n'est pas limitée à ce qui vient d'être décrit en relation avec les figures.

Par exemple, l'écran et le système de visualisation directement sur le corps peuvent être remplacés par un casque de réalité virtuelle ou augmentée.

La caméra thermique peut être remplacée par un capteur de température ou de la couleur de la peau.

L'opérateur peut être remplacé par un bras robotisé, éventuellement commandé directement par le dispositif. Un opérateur peut alors rentrer les informations décrites précédemment et le bras robotisé effectue le traitement. L'opérateur peut suivre à l'écran l'évolution du traitement et l'arrêter à tout moment.

Dans ce qui précède, un traitement d'épilation est décrit. Cependant un tel dispositif peut être utilisé dans le cadre d'un traitement de photo-rajeunissement ou d'un traitement vasculaire, notamment au prix de quelques ajustements liés aux caractéristiques de chacun de ces traitements. Les longueurs d'onde des diodes laser sont de préférence entre 500 et 530 nm pour un dispositif de traitement vasculaire.

Dans ce qui précède, un dispositif de traitement à rayonnement laser est décrit. Cependant l'invention n'est pas limitée à un tel exemple et un dispositif LPP ou IPL peut être utilisé.

## Revendications

1. Dispositif (10) d'épilation, de photorajeunissement et/ou de traitement vasculaire d'une partie (P) d'un corps humain (H) par émission d'impulsions lumineuses, comportant :
- une pièce à main (30) pour appliquer les impulsions lumineuses sur la partie du corps (P), la pièce à main (30) étant déplaçable par rapport au corps et comportant une fenêtre (32) de sortie des impulsions lumineuses sur une zone localisée (33) de la partie du corps, et
- au moins un système de projection (90, 92) pour projeter une information de guidage sur la partie du corps (P) afin de guider l'opérateur durant le traitement et/ou une information d'aide à la localisation de la pièce à main et/ou du corps, **caractérisé en ce que** le système de projection (90, 92) éclaire au moins partiellement la partie du corps à traiter pour différencier les zones traitées des zones non traitées de la partie du corps à traiter.

2. Dispositif selon la revendication 1, dans lequel les zones traitées sont différenciées des zones non traitées par une différence de luminosité, couleur ou éclairage.

3. Dispositif selon la revendication 1 ou 2, dans lequel le système de projection (90, 92) produit une image sur la partie du corps configurée pour différencier, notamment par une différence de luminosité, couleur, les zones de la peau par le nombre de passages de la pièce à main (30) qu elles ont subi.

4. Dispositif selon l'une quelconque des revendications précédentes, dans lequel la projection de l'information sur la zone de la partie du corps (P) par le système de projection (90, 92) est ajustée en temps réel en fonction des mouvements du corps.

5. Dispositif selon l'une quelconque des revendications précédentes, dans lequel les impulsions lumineuses sont en une lumière polychromatique pulsée, notamment une lumière intense pulsée ou des impulsions laser.

6. Dispositif selon l'une quelconque des revendications précédentes, comportant deux systèmes de projection (90, 92) orientées selon des directions de visée différentes par rapport à la partie du corps à traiter (P).

7. Dispositif selon l'une quelconque des revendications précédentes, dans lequel le système de projection (90, 92) projette des franges sur la partie du corps pour aider à la détermination des mouvements de la partie du corps, de préférence, le dispositif comportant une caméra (12, 14) d'analyse des franges projetées sur la peau.

8. Dispositif selon l'une quelconque des revendications précédentes, comportant au moins une caméra (12, 14), de préférence au moins deux caméras (12, 14), disposées pour avoir dans son champ de vision la pièce à main, notamment un champ de vision qui englobe l'intégralité de la partie du corps (P) à traiter, et un système de traitement des images fournies par la ou les caméras (12, 14) pour localiser la pièce à main (30) par rapport à la partie du corps (P) à partir au moins de ces images et optionnellement des informations fournies par l'analyse des franges sur la peau.

9. Dispositif selon l'une quelconque des revendications précédentes, comportant un détecteur de température (16), notamment une caméra thermique, pour contrôler la température locale de la zone exposée à l'impulsion lumineuse, et un système de commande (23) configuré pour bloquer le déclenchement des impulsions lumineuses ou émettre un signal d'alerte pour l'opérateur lorsque la température locale est supérieure à une température limite, le système de commande (23) étant de préférence configuré pour déterminer les zones traitées de la partie du corps à traiter à l'aide des informations fournies par le détecteur de température (16) depuis le début du traitement et optionnellement des informations fournies par l'analyse des franges sur la peau

10. Dispositif selon l'une quelconque des revendications précédentes, dans lequel les impulsions lumineuses sont des impulsions laser et le dispositif comporte un poste de base (20) comportant une source laser émettant des impulsions laser, la pièce à main étant reliée optiquement à la source laser par au moins un guide optique (40), le poste de base (20) comportant de préférence une pluralité de diodes laser disposées selon une pluralité de rangées (24), les diodes d'une même rangée de diodes laser (24) étant montées en série et les différentes rangées de diodes laser (24) étant montées en parallèle, ou commandées indépendamment de préférence, chaque rangée de diodes laser (24) présentant des diodes laser émettant toutes soit à environ 750 nm, soit à environ 1064 nm, et optionnellement une ou plusieurs diodes laser émettant dans le visible, un système de commande (23) du dispositif déterminant de préférence la séquence de déclenchement des diodes laser (24), notamment les rangées de diodes laser (24) à déclencher, les intensités de chacune des rangées de diodes laser (24), la durée de déclenchement de chacune des rangées de diodes laser (24), et/ou le moment de déclenchement de chaque rangée de diodes laser (24), en fonction d'au moins une caractéristique de la peau, notamment le phototype de la peau, la caractéristique de la peau étant de préférence déterminée à partir des informations fournies par le détecteur de mouvement (34) et/ou à partir de données choisies par l'opérateur.

11. Dispositif selon l'une quelconque des revendications précédentes, comportant au moins une caméra (12, 14), de préférence au moins deux caméras (12, 14), disposées pour avoir chacune dans son champ de vision la pièce à main (30), de préférence un champ de vision qui englobe l'intégralité de la partie du corps (P) à traiter, et un système de traitement des images fournies par la ou les caméras (12, 14) pour localiser la pièce à main (30) par rapport à la partie du corps (P) à partir au moins de ces images.

12. Dispositif selon l'une quelconque des revendications précédentes, comportant un système de visualisation (50) du traitement, notamment un écran (50), configuré pour afficher une image de la partie du corps (P), notamment une image tridimensionnelle, et optionnellement une ou plusieurs des caractéristiques suivantes :
- la position de la pièce à main (30) sur le corps fournie par la ou les caméras (12, 14),
- les zones de la partie du corps (P) traitées et/ou les zones de la partie du corps (P) non traitées, et/ou
- l'historique de température de la peau à partir des informations fournies par le détecteur de température (16) depuis le début du traitement en chaque point de la partie du corps (P) à traiter.

13. Dispositif selon l'une quelconque des revendications précédentes, dans lequel chaque impulsion lumineuse émise par la fenêtre de sortie (32) présente une puissance surfacique supérieure ou égale à 200 W/cm², de préférence supérieure ou égale à 1 kW/cm², mieux supérieure ou égale à 2 kW/cm².

14. Procédé esthétique de traitement d'une partie d'un corps par émission d'impulsions lumineuses à l'aide d'un dispositif selon l'une quelconque des revendications précédentes, comportant les étapes consistant à projeter sur la partie du corps l'information de guidage ou d'aide à la localisation de la pièce à main (30) avec le système de projection (90, 92) et à traiter la partie du corps (P) en fonction de l'information de guidage et/ou à localiser la pièce à main et/ou le corps.

15. Procédé selon la revendication 14, comportant une étape d'application de la pièce à main (30) sur la partie du corps à traiter et une étape de balayage de la partie du corps à traiter avec la pièce à main (30), notamment en maintenant une pression avec la pièce à main (30) sur la peau.

## Patentansprüche

1. Vorrichtung (10) zur Enthaarung, zur Photoverjüngung und/oder zur Gefäßbehandlung eines Teils (P) eines menschlichen Körpers (H) durch Emission von Lichtpulsen, umfassend
- ein Handstück (30), um die Lichtpulse auf den Teil des Körpers (P) anzuwenden, wobei das Handstück (30) in Bezug auf den Körper bewegbar ist und ein Fenster (32) für das Austreten der Lichtpulse auf einen lokalisierten Bereich (33) des Teils des Körpers umfasst, und
- mindestens ein Projektionssystem (90, 92), um auf den Teil des Körpers (P) eine Leitinformation, um den Bediener während der Behandlung zu leiten, und/oder eine Hilfsinformation zur Lokalisierung des Handstücks und/oder des Körpers zu projizieren, **dadurch gekennzeichnet, dass** das Projektionssystem (90, 92) den zu behandelnden Teil des Körpers mindestens teilweise beleuchtet, um die behandelten Bereiche von den nicht behandelten Bereichen des zu behandelnden Teils des Körpers zu unterscheiden.

2. Vorrichtung nach Anspruch 1, wobei die behandelten Bereiche von den nicht behandelten Bereichen durch einen Unterschied in der Helligkeit, der Farbe oder der Beleuchtung unterschieden werden.

3. Vorrichtung nach Anspruch 1 oder 2, wobei das Projektionssystem (90, 92) ein Bild auf dem Teil des Körpers produziert, das dazu konfiguriert ist, insbesondere durch einen Unterschied in der Helligkeit, der Farbe, die Bereiche der Haut gemäß der Anzahl von Durchläufen des Handstücks (30), die sie erfahren haben, zu unterscheiden.

4. Vorrichtung nach einem beliebigen der vorhergehenden Ansprüche, wobei die Projektion der Information auf den Bereich des Teils des Körpers (P) durch das Projektionssystem (90, 92) in Abhängigkeit von den Bewegungen des Körpers in Echtzeit angepasst wird.

5. Vorrichtung nach einem beliebigen der vorhergehenden Ansprüche, wobei die Lichtpulse aus einem gepulsten polychromatischen Licht, insbesondere einem intensiv gepulsten Licht oder Laserpulsen, bestehen.

6. Vorrichtung nach einem beliebigen der vorhergehenden Ansprüche, die zwei Projektionssysteme (90, 92) umfasst, die in Bezug auf den zu behandelnden Teil des Körpers (P) gemäß unterschiedlichen Betrachtungsrichtungen ausgerichtet sind.

7. Vorrichtung nach einem beliebigen der vorhergehenden Ansprüche, wobei das Projektionssystem (90, 92) Streifen auf den Teil des Körpers projiziert, um bei der Bestimmung der Bewegungen des Teils des Körpers zu helfen, wobei die Vorrichtung vorzugsweise eine Kamera (12, 14) zur Analyse der auf die Haut projizierten Streifen umfasst.

8. Vorrichtung nach einem beliebigen der vorhergehenden Ansprüche, umfassend mindestens eine Kamera (12, 14), vorzugsweise mindestens zwei Kameras (12, 14), die so angeordnet sind, dass sich in ihrem Sichtfeld das Handstück befindet, insbesondere ein Sichtfeld aufweisen, das die Gesamtheit des zu behandelnden Teils des Körpers (P) einschließt, und ein System zur Verarbeitung der Bilder, die durch die eine oder die mehreren Kameras (12, 14) bereitgestellt werden, um das Handstück (30) in Bezug auf den Teil des Körpers (P) anhand mindestens dieser Bilder und optional der Informationen, die durch die Analyse der Streifen auf der Haut bereitgestellt werden, zu lokalisieren.

9. Vorrichtung nach einem beliebigen der vorhergehenden Ansprüche, umfassend einen Temperatursensor (16), insbesondere eine Wärmekamera, um die lokale Temperatur des Bereichs, der dem Lichtpuls ausgesetzt ist, zu überwachen, und ein Steuersystem (23), das dazu konfiguriert ist, die Auslösung der Lichtpulse zu blockieren oder ein Warnsignal für den Bediener zu emittieren, wenn die lokale Temperatur höher als eine Grenztemperatur ist, wobei das Steuersystem (23) vorzugsweise dazu konfiguriert ist, die behandelten Bereiche des zu behandelnden Teils des Körpers mit Hilfe der Informationen, die durch den Temperatursensor (16) seit Beginn der Behandlung bereitgestellt werden, und optional der Informationen, die durch die Analyse der Streifen auf der Haut bereitgestellt werden, zu bestimmen.

10. Vorrichtung nach einem beliebigen der vorhergehenden Ansprüche, wobei die Lichtpulse Laserpulse sind und die Vorrichtung eine Basisstation (20) umfasst, die eine Laserquelle umfasst, die Laserpulse emittiert, wobei das Handstück durch mindestens einen Lichtleiter (40) mit der Laserquelle optisch verbunden ist, wobei die Basisstation (20) vorzugsweise eine Vielzahl von Laserdioden umfasst, die gemäß einer Vielzahl von Reihen (24) angeordnet sind, wobei die Dioden einer gleichen Reihe von Laserdioden (24) in Reihe geschaltet sind und die unterschiedlichen Reihen von Laserdioden (24) parallel geschaltet sind oder vorzugsweise unabhängig gesteuert werden, wobei jede Laserdiodenreihe (24) Laserdioden, die alle entweder bei ungefähr 750 nm oder bei ungefähr 1064 nm emittieren, und optional eine oder mehrere Laserdioden, die im sichtbaren Bereich emittieren, aufweist, wobei ein Steuersystem (23) der Vorrichtung vorzugsweise die Auslösereihenfolge der Laserdioden (24), insbesondere die auszulösenden Laserdiodenreihen (24), die Intensitäten jeder der Laserdiodenreihen (24), die Auslösedauer jeder der Laserdiodenreihen (24) und/oder den Moment der Auslösung jeder Laserdiodenreihe (24) in Abhängigkeit von mindestens einer Eigenschaft der Haut, insbesondere dem Phototyp der Haut, bestimmt, wobei die Eigenschaft der Haut vorzugsweise anhand der Informationen, die durch den Bewegungssensor (34) bereitgestellt werden, und/oder anhand von Daten, die durch den Bediener ausgewählt werden, bestimmt wird.

11. Vorrichtung nach einem beliebigen der vorhergehenden Ansprüche, umfassend mindestens eine Kamera (12, 14), vorzugsweise mindestens zwei Kameras (12, 14), die so angeordnet sind, dass sich in ihrem Sichtfeld jeweils das Handstück (30) befindet, vorzugsweise ein Sichtfeld aufweisen, das die Gesamtheit des zu behandelnden Teils des Körpers (P) einschließt, und ein System zur Verarbeitung der Bilder, die durch die eine oder die mehreren Kameras (12, 14) bereitgestellt werden, um das Handstück (30) in Bezug auf den Teil des Körpers (P) anhand mindestens dieser Bilder zu lokalisieren.

12. Vorrichtung nach einem beliebigen der vorhergehenden Ansprüche, umfassend ein Visualisierungssystem (50) der Behandlung, insbesondere einen Bildschirm (50), das dazu konfiguriert ist, ein Bild des Teils des Körpers (P), insbesondere ein dreidimensionales Bild, und optional eine oder mehrere der folgenden Eigenschaften anzuzeigen:
- die Position des Handstücks (30) auf dem Körper, die durch die eine oder die mehreren Kameras (12, 14) bereitgestellt wird,
- die behandelten Bereiche des Teils des Körpers (P) und/oder die nicht behandelten Bereiche des Teils des Körpers (P) und/oder
- den Temperaturverlauf der Haut anhand der Informationen, die seit Beginn der Behandlung an jedem Punkt des zu behandelnden Teils des Körpers (P) durch den Temperatursensor (16) bereitgestellt werden.

13. Vorrichtung nach einem beliebigen der vorhergehenden Ansprüche, wobei jeder durch das Austrittsfenster (32) emittierte Lichtpuls eine Wärmestromdichte größer als oder gleich 200 W/cm², vorzugsweise größer als oder gleich 1 kW/cm², noch bevorzugter größer als oder gleich 2 kW/cm² aufweist.

14. Ästhetisches Verfahren zur Behandlung eines Teils eines Körpers durch Emission von Lichtpulsen mit Hilfe einer Vorrichtung nach einem beliebigen der vorhergehenden Ansprüche, umfassend die Schritte, die darin bestehen, mit dem Projektionssystem (90, 92) auf den Teil des Körpers (P) die Leitinformation oder die Hilfsinformation zur Lokalisierung des Handstücks (30) zu projizieren und den Teil des Körpers (P) in Abhängigkeit von der Leitinformation zu behandeln und/oder das Handstück und/oder den Körper zu lokalisieren.

15. Verfahren nach Anspruch 14, umfassend einen Schritt des Anwendens des Handstücks (30) auf den zu behandelnden Teil des Körpers und einen Schritt des Überstreichens des zu behandelnden Teils des Körpers mit dem Handstück (30), insbesondere indem mit dem Handstück (30) ein Druck auf die Haut aufrechterhalten wird.

## Claims

1. Device (10) for removing hair from, photorejuvenating and/or vascular treatment of part (P) of a human body (H) by emitting light pulses, comprising:
- a handpiece (30) for applying the light pulses to the part (P) of the body, the handpiece (30) being able to move in relation to the body and comprising a window (32) for outputting the light pulses onto a localized area (33) of the part of the body, and
- at least one projection system (90, 92) for projecting guidance information onto the part (P) of the body in order to guide the operator during the treatment and/or information for assisting with the location of the handpiece and/or the body, **characterized in that** the projection system (90, 92) at least partially illuminates the part of the body to be treated in order to distinguish between the treated areas and the untreated areas of the part of the body to be treated.

2. Device according to Claim 1, wherein the treated areas are distinguished from the untreated areas through a difference in brightness, colour or illumination.

3. Device according to Claim 1 or 2, wherein the projection system (90, 92) produces an image on the part of the body that is configured so as to distinguish, in particular through a difference in brightness or colour, between the areas of the skin in terms of the number of passes of the handpiece (30) that they have experienced.

4. Device according to any one of the preceding claims, wherein the projection of the information onto the area of the part (P) of the body by the projection system (90, 92) is adjusted in real time on the basis of the movements of the body.

5. Device according to any one of the preceding claims, wherein the light pulses are composed of polychromatic pulsed light, in particular intense pulsed light or laser pulses.

6. Device according to any one of the preceding claims, comprising two projection systems (90, 92) oriented in different viewing directions with respect to the part (P) of the body to be treated.

7. Device according to any one of the preceding claims, wherein the projection system (90, 92) projects fringes onto the part of the body in order to help determine the movements of the part of the body, the device preferably comprising a camera (12, 14) for analysing the fringes projected onto the skin.

8. Device according to any one of the preceding claims, comprising at least one camera (12, 14), preferably at least two cameras (12, 14), arranged so as to have the handpiece in its field of view, in particular a field of view that encompasses the entire part (P) of the body to be treated, and a processing system for processing the images provided by the camera or cameras (12, 14) in order to locate the handpiece (30) with respect to the part (P) of the body from at least these images and optionally the information provided by analysing the fringes on the skin.

9. Device according to any one of the preceding claims, comprising a temperature detector (16), in particular a thermal camera, for monitoring the local temperature of the area exposed to the light pulse, and a control system (23) configured so as to block triggering of the light pulses or to emit an alert signal for the operator when the local temperature is greater than a limit temperature, the control system (23) preferably being configured so as to determine the treated areas of the part of the body to be treated using the information provided by the temperature detector (16) since the start of the treatment and optionally the information provided by analysing the fringes on the skin.

10. Device according to any one of the preceding claims, wherein the light pulses are laser pulses and the device comprises a base station (20) comprising a laser source emitting laser pulses, the handpiece being optically connected to the laser source by at least one optical guide (40), the base station (20) preferably comprising a plurality of laser diodes arranged in a plurality of rows (24), the diodes in one and the same row of laser diodes (24) being connected in series and the various rows of laser diodes (24) being connected in parallel, or preferably controlled independently, each row of laser diodes (24) having laser diodes all emitting either at around 750 nm or at around 1064 nm, and optionally one or more laser diodes emitting in the visible, a control system (23) of the device preferably determining the trigger sequence of the laser diodes (24), in particular the rows of laser diodes (24) to be triggered, the intensities of each of the rows of laser diodes (24), the trigger duration of each of the rows of laser diodes (24), and/or the trigger time of each row of laser diodes (24), on the basis of at least one feature of the skin, in particular the phototype of the skin, the feature of the skin preferably being determined from the information provided by the motion detector (34) and/or from data chosen by the operator.

11. Device according to any one of the preceding claims, comprising at least one camera (12, 14), preferably at least two cameras (12, 14), each arranged so as to have the handpiece (30) in its field of view, preferably a field of view that encompasses the entire part (P) of the body to be treated, and a processing system for processing the images provided by the camera or cameras (12, 14) in order to locate the handpiece (30) with respect to the part (P) of the body from at least these images.

12. Device according to any one of the preceding claims, comprising a treatment viewing system (50), in particular a screen (50), configured so as to display an image of the part (P) of the body, in particular a three-dimensional image, and optionally one or more of the following features:
- the position of the handpiece (30) on the body as provided by the camera or cameras (12, 14),
- the treated areas of the part (P) of the body and/or the untreated areas of the part (P) of the body, and/or
- the history of the temperature of the skin based on the information provided by the temperature detector (16) since the start of the treatment at each point of the part (P) of the body to be treated.

13. Device according to any one of the preceding claims, wherein each light pulse emitted by the output window (32) has a power flux density greater than or equal to 200 W/cm², preferably greater than or equal to 1 kW/cm², better still greater than or equal to 2 kW/cm².

14. Aesthetic method for treating a part of a body by emitting light pulses using a device according to any one of the preceding claims, comprising the steps of projecting, onto the part of the body, guidance information or information for assisting with the location of the handpiece (30) with the projection system (90, 92) and of treating the part (P) of the body on the basis of the guidance information and/or of locating the handpiece and/or the body.

15. Method according to Claim 14, comprising a step of applying the handpiece (30) to the part of the body to be treated and a step of scanning the part of the body to be treated with the handpiece (30), in particular while keeping the handpiece (30) pressed on the skin.
